# EUROPEAN PATENT SPECIFICATION

(11) **EP 3 215 064 B1**
(45) Date of publication and mention of the grant of the patent: **04.03.2020**
(21) Application number: 15828778.9
(22) Date of filing: 05.11.2015
(51) Int. Cl.: A61F 2/36, A61F 2/38, A61F 2/30

(54) **MOULD FOR FORMING OF A JOINT SPACER DEVICE OR A PART THEREOF**
FORM ZUM GIESSEN EINES GELENKDISTANZHALTERS ODER EINES TEILS DAVON
MOULE A FORMER UN ESPACEUR D'ARTICULATION OU D'UNE PARTIE DE CELA

(30) Priority: 06.11.2014 IT RM20140645
(43) Date of publication of application: 13.09.2017
(73) Proprietor: Cossington Limited, 167-169 London Road Kingston Upon Thames Surrey KT2 6PT (GB)
(72) Inventor: Cappelletti, Ava, 47521 Cesena (IT)
(74) Representative: Dehns
(86) International application number: PCT/IT2015/000267
(87) International publication number: WO 2016/071939

(56) References cited:
- US-A1- 2010 102 484
- US-A1- 2013 344 186
- US-B1- 6 361 731

## Description

### TECHNICAL FIELD OF THE INVENTION

This invention relates to a mould for forming a joint spacer device or a part thereof, as described in the claims.

### STATE OF THE ART

At the state of the art moulds for joint spacer devices are known, which are usually made in elastically deformable material, such as, for example, silicone or other elastomeric suitable material, and intended to be filled with bone cement supplemented with antibiotic or other therapeutic substance, when the latter is in the fluid and malleable mass phase, for the purpose of obtaining a finished spacer device.

The bone cement supplemented with antibiotic or other therapeutic substance, after having hardened in such a mould, is subsequently removed therefrom and the joint spacer thus obtained, once finished off, is implanted in the joint space of a patient to treat a disease in progress in the tissues surrounding such joint space.

The traditional moulds suffer from several drawbacks.

In the first place, being these made in elastically yielding-deformable material, such as silicone, they do not allow obtaining a spacer device with a definite shape in optimal manner, for which reason, once extracted from the mould, a spacer device must be machined superficially and finished, in order to obtain the exact required shape.

These moulds, furthermore, although being in most cases expected to be disposed of/thrown away after only one single use, at times are reused after an appropriate washing process, for the purpose of reducing the costs for obtaining spacer devices having the same shape.

It can happen, however, that such a washing does not remove completely from the mould the traces of the antibiotic or additive therapeutic substance with bone cement used for a previous spacer device, with the result that allergic reactions may occur in spacer devices made with an antibiotic to which a patient is not allergic, which was, however, made in a mould used previously with bone cement supplemented with an antibiotic to which the patient is allergic US 2010/102484 discloses a mold for forming a joint spacer device comprising a rigid container body and a rigid cover provided with a first and a second perimeter profile delimiting a first and a second moulding surface intended to shape a first and a second portion of said joint spacer, respectively, said at least one rigid container body and said at least one rigid cover being engageable in a removable manner to each other so as to delimit a cavity corresponding to the external configuration of said joint spacer or said part thereof and said mould comprising weakening means on said at least one rigid container body and cover so that both said body and said cover are separable into parts to allow the extraction of said at least one spacer device or said at least one part thereof moulded between them.

### OBJECTS OF THE INVENTION

The mould of the invention is described in the claims.

Therefore, it is the main object of the present invention to improve the state of the art in the industry sector of moulds for the formingof joint spacer devices or a part thereof.

It is another object of the present invention to provide a mould for the forming of a joint spacer device or a part thereof with an alternative configuration with respect to traditional moulds.

It is another object of the present invention to provide a mould for the forming of a joint spacer device or a part thereof that cannot be used more than once.

It is a further object of the present invention to provide a mould for the forming of a joint spacer device or a part thereof, which does not require laborious finishing steps of the spacer extracted thereof.

It is still another object of the present invention to provide a mould for the forming of a joint spacer device or a part thereof, which is easy to make at competitive costs.

Not the least object of the present invention is that of providing a joint spacer device or a part thereof obtained by means of such a mould.

According with an aspect of the present invention a mould is provided for forming a joint spacer device or a part thereof according to claim 1.

Dependent claims refer to preferred and advantageous embodiments of the invention.

### BRIEF DESCRIPTION OF THE DRAWINGS

Further characteristics and advantages of the present invention will become more evident from the detailed description of some examples of preferred embodiments of a mould for forming a joint spacer device or a part thereof, shown and described by way of example, but not limited thereto, in the attached table of drawings wherein:
Figure 1 is a plan view of a mould for forming a joint spacer device for the hip-joint according to a first embodiment of this invention;
Figure 2 shows a rear view of the mould of Figure 1;
Figure 3 illustrates a perspective view in side elevation of the mould of Figure 1;
Figure 4 is a perspective view in side elevation of the mould of Figure 1 in an intermediate position between an opening position and a closing position;
Figure 5 shows a detail of the mould of Figure 1;
Figure 6 is a plan view of a mould for forming a part of a joint spacer device for the joint of the knee according to a second embodiment of the present invention;
Figure 7 shows a perspective view of the mould cover of Figure 6;
Figure 8 illustrates a perspective view of the mould container body of Figure 6;
Figure 9 is a perspective view of the mould of Figure 6 with container body and cover engaged with each other thereby delimiting a forming seat;
Figure 10 shows another perspective view of the mould of Figure 6, wherein container body and cover are engaged with each other;
Figure 11 illustrates a perspective view in side elevation of a mould for forming a part of a joint spacer device according to a third embodiment of this invention;
Figure 12 shows a plan view of the mould of Figure 11;
Figure 13 illustrates a perspective view of the container body of the mould of Figure 11;
Figure 14 is a perspective view of the cover of the mould of Figure 11;
Figure 15 shows a perspective view in front elevation of the mould of Figure 11, wherein the container body and the cover are in intermediate position with the cover partly inserted in the container; and
Figure 16 illustrates a perspective view of the mould of Figure 11, wherein the cover closes the container body and delimits with the latter the forming seat.

In the attached drawings equal parts or components are marked by the same reference numerals.

### MODES OF CARRYING OUT THE INVENTION

With reference to the attached figures, a mould according to this disclosure is generally indicated with number 1. Mould 1, according to the present disclosure, is made in elastically undeformable plastic material, for example polyethylene, and, preferably, distinguishable by a high Shore A, comprised between 90 and 150.

Mould 1, comprises at least a rigid container body 2 and at least a rigid cover 3, which can be coupled with each other to delimit a cavity 4 corresponding to an external configuration of a joint spacer to be formed or a part thereof.

More particularly, the rigid container body 2 is provided with at least a first perimeter profile 5, which internally delimits at least a first moulding surface 6 intended to shape at least a first part of the joint spacer device.

The rigid cover 3 is also provided with at least a second perimeter profile 7, delimiting at least a second moulding surface 8 intended to shape at least a second part of such joint spacer device.

The rigid container body 2 and the rigid cover 3 engage with each other in a removable manner at the respective perimeter profiles 5 and 7, so that the cavity 4 remains circumscribed therebetween.

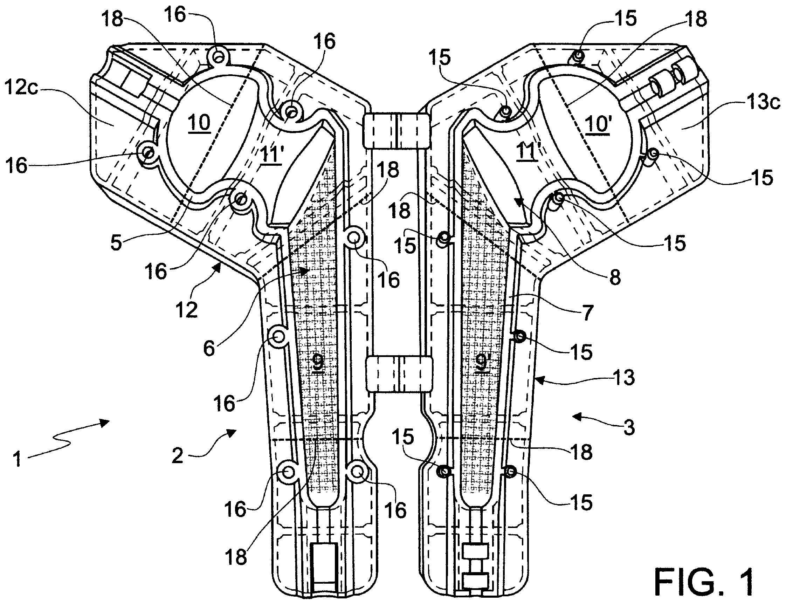

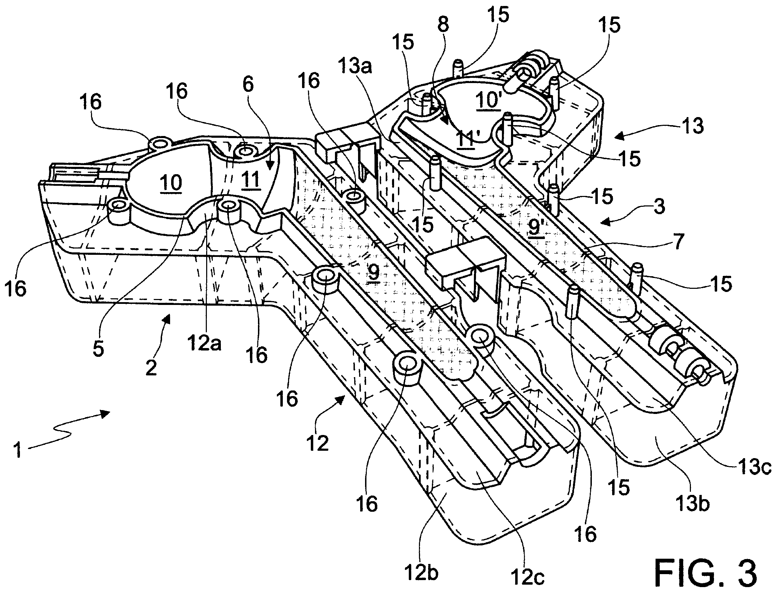

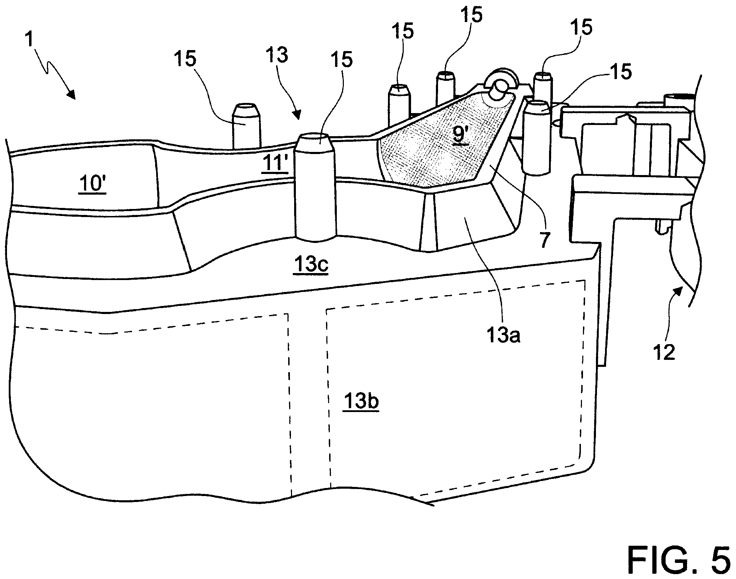

With particular reference to Figures 1 to 5, which illustrate a mould for forming a joint spacer device according to a first embodiment of this disclosure, it will be noted that, in such a mould, the perimeter profile 5 of the rigid container body 2 circumscribes a forming surface for a longitudinal half of a joint spacer device for hip, and the perimeter profile 7 of the rigid cover 3 circumscribes a second moulding surface 8 for the other longitudinal half of such a spacer device.

Each moulding surface 6 and 8 comprises a stem portion 9, 9', a head portion 10, 10', and a connection portion 11, 11' between the stem portion and the head portion.

In the mould according to the first embodiment of the present disclosure the perimeter profiles 5 and 7 coincide with the access opening to the container body 2 and the cover 3 , respectively. across the access opening it is possible to cast the bone cement supplemented with a suitable antibiotic, pouring it on the first moulding surface 6 and on the second moulding surface 8, so as to cover them up to the level of the respective perimeter profile 5, 7.

According to a variation of the mould according to the disclosure, the moulding surfaces 6 and 8 are each obtained in a respective support base 12, 13, and each perimeter profile 5 and 7 extends substantially orthogonally from the respective base and away from it. Between the upper end of each perimeter profile 5, 7 and the support base 12, 13 the mould according to the present disclosure comprises at least an inclined plane perimeter wall 12a, 13a. The inclined plane perimeter walls 12a, 13a of the rigid container body 2 and of the rigid cover 3, besides carrying out a stiffening function of the respective perimeter profile 5, 7, facilitate, in addition, the disposal of the bone cement poured in excess on the moulding surfaces 6 and/or 8 during the forming stage.

The mould according to the disclosure, as already mentioned above, is configured in such a manner that, once the bone cement is poured on the moulding surfaces 6, 8 and after a certain time has passed for the partial hardening thereof, the rigid container body 2 and rigid cover 3 are engaged with each other and delimit a cavity 4 of finite size. The bone cement therefore, which can be accidentally poured in excess on the moulding surfaces 6 and/or 8, outflows sideways at the perimeter sections and it separates distinctly from the bone cement contained in the cavity 4, thanks to the inclined plane perimeter walls 12a and 13a that facilitate its removal. It follows that, once the bone cement has completely hardened and it has been separated from the mould, as it will be better said hereinafter, the hip spacer device thus obtained comprises a minimum perimeter edge (of width equal to the maximum of that of the perimeter profiles 5 and 7) and requires, therefore, minimum finishing work before the implantation in situ.

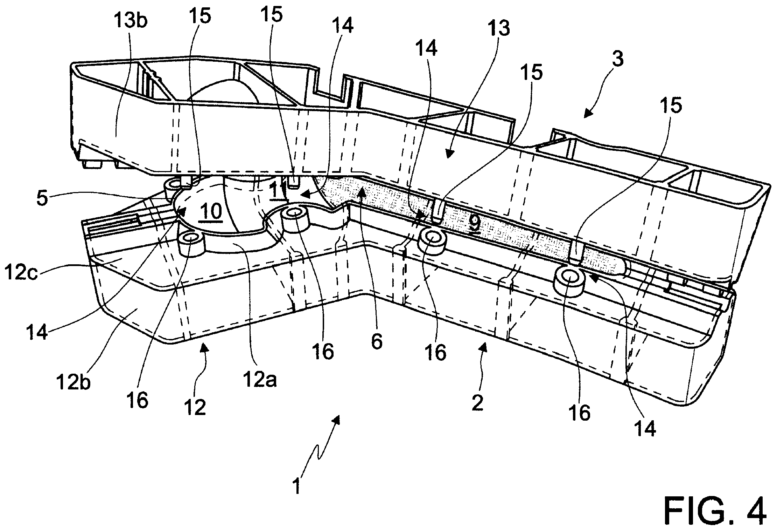

For the removable type engagement between the rigid container body 2 and the rigid cover 3, the mould according to the first embodiment of this disclosure comprises removable engagement means 14 of the male 15 and female 16 types. Such removable engagement means 14 are provided in positions at, and along, each perimeter profile 5, 7, so that, when the rigid container body 2 and the respective rigid cover 3 engage with each other at their respective perimeter profiles 5 and 7, the engagement between the male engagement means 15 and the female engagement means 16 of the mould (see, in particular, Figure 4) is also achieved. According to a variation illustrated in the figures of the first embodiment of this disclosure, the rigid container body 2 and the rigid cover 3 are hinged with each other at the respective bases 12, 13.

Each base 12, 13 can, furthermore, comprise a solid body made of suitable plastic material as described above, wherein the respective moulding surface 6, 8 is hollowed out. Alternatively, it can comprise side walls 12b, 13b, delimiting at the top a base surface 12c, 13c from which the respective perimeter profile 5, 7 of the rigid container body 2 and the rigid cover 3 rise. In this case, each base 12 and/or 13 can comprise, between the side walls, lower with respect to the rigid container body 2 and/or the cover 3, one or more transversal stiffening ribs 17, so that the mould 1 according to the disclosure is elastically undeformable although being made with a reduced quantity of material.

Now, the mould 1 according to the first embodiment of the present disclosure comprises weakening means 18 on the rigid container body 2 and on the rigid cover 3, so that both the container body and the cover can be separated in several parts in order to allow extraction of the spacer device formed therein.

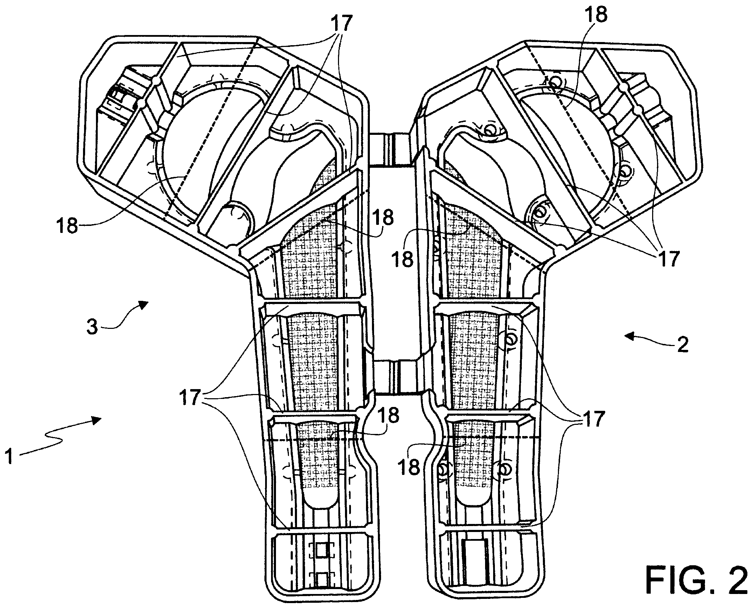

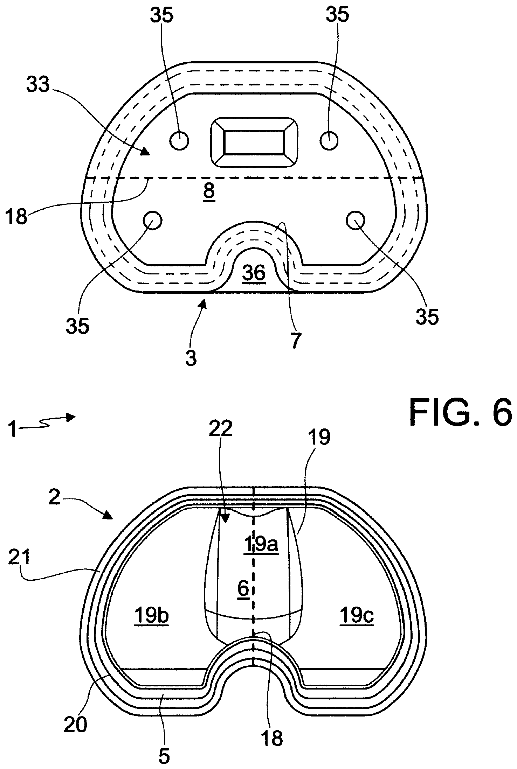

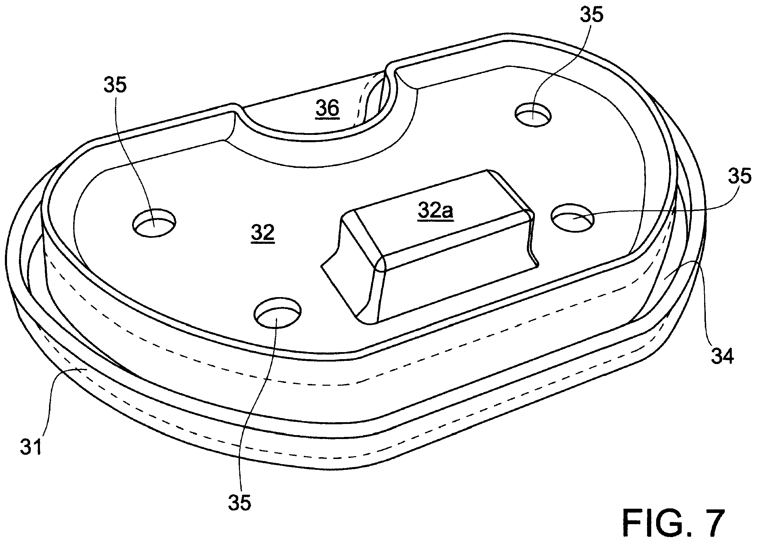

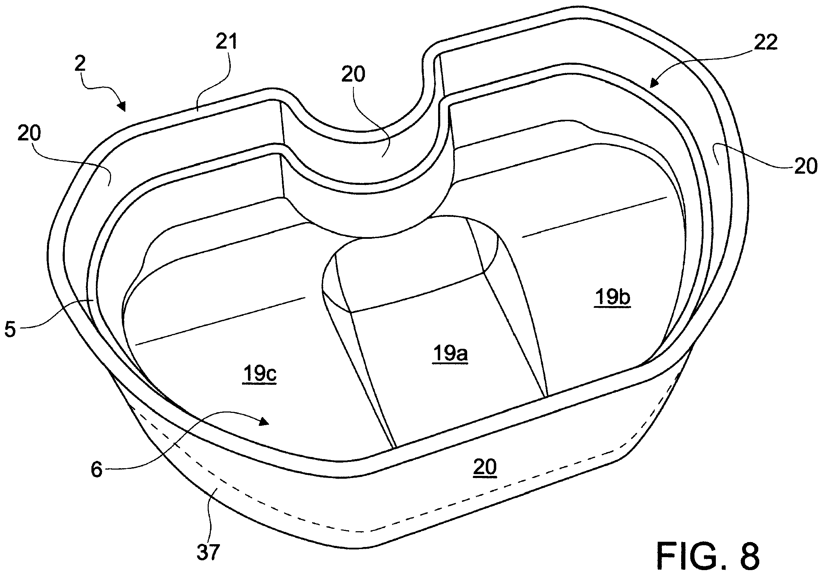

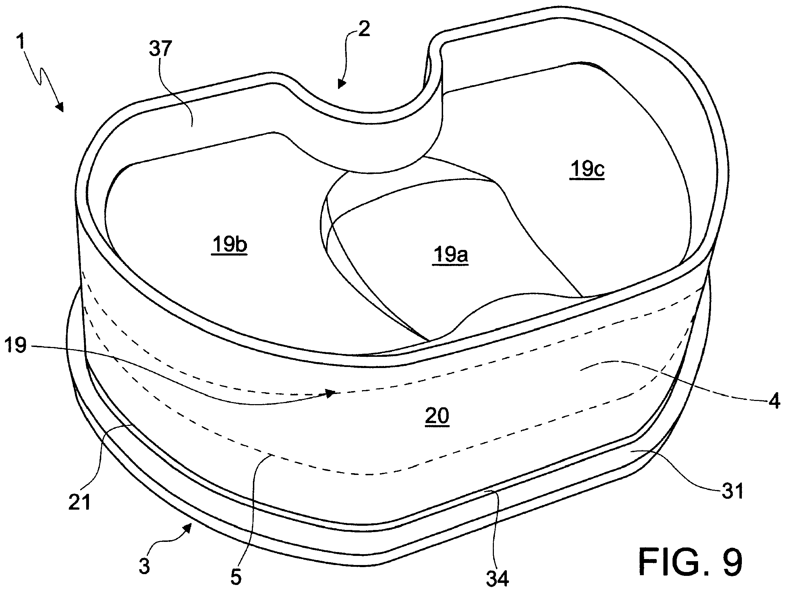

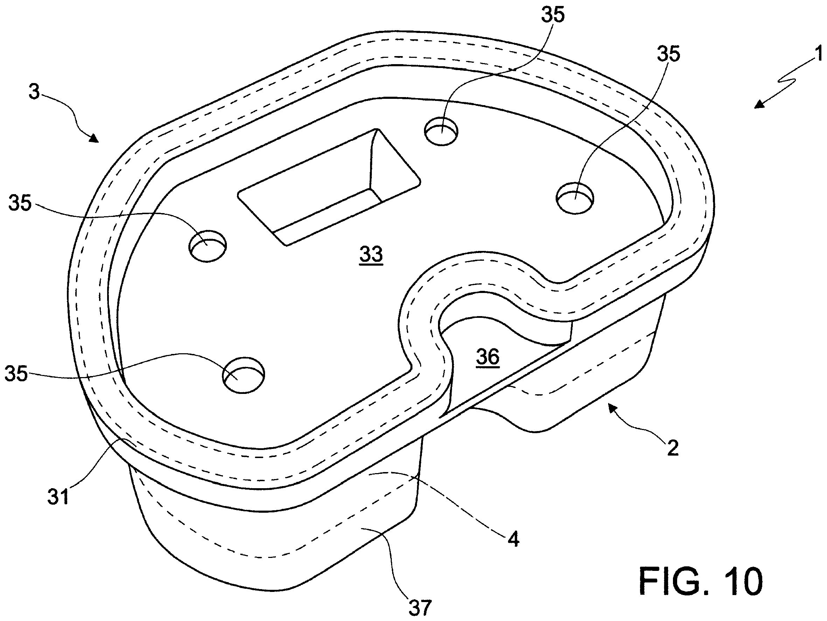

Such weakening means, illustrated schematically in the drawings, comprise at least a reduced thickness line (indicated merely by way of example in Figures 1 and 2 with dashed lines), at which the rigid container body 2 and the rigid cover 3 can be separated in several parts. Such a reduced thickness line is provided transversally and/or longitudinally on both the rigid container body 2 and the rigid cover 3. The mould 1 according to a second embodiment of the present disclosure, see in particular the annexed Figures 6 to 10, is particularly suitable for forming a tibial portion of a spacer device for knee joint and comprises at least a rigid container body 2 and at least a rigid cover 3, which can be coupled with each other to delimit a cavity 4 corresponding to the external configuration of such component or tibial part.

More particularly, the rigid container body 2 of the mould according to the disclosure has a substantially "C"-shaped plan configuration and comprises a bottom wall 19 from which side walls 20 rise. The side walls 20 end at the top with an edge 21, at an upper access opening 22.

The rigid container body 2 is provided with at least a first perimeter profile 5, which upperly delimits at least a first moulding surface 6 intended to shape at least a first part of the joint spacer device.

The first perimeter profile 5 is defined by an internal shoulder at the side walls 20, at a distance substantially constant from the edge 21.

Also the rigid cover 3 has a substantially "C"-shaped plan configuration and is provided with at least a second perimeter profile 7, delimiting at least a second moulding surface 8, intended to shape at least a second part of such joint spacer device.

The rigid container body 2 and the rigid cover 3 are engageable with each other in a removable manner at their respective perimeter profiles 5 and 7, so that the cavity 4 remains delimited therebetween.

As it will be noted, the bottom wall 19 of the rigid container body 2 of the mould 1 according to the second embodiment of the present disclosure is substantially convex and has a central portion 19a and two end portions 19b and 19c, at the free ends of the "C". The central portion 19a is substantially in bas-relief with respect to the end portions 19b and 19c.

Now, with reference to the rigid cover 3 of the mould of Figures 6 to 10, it will be noted how this has a lower surface 32, facing, in use, the respective rigid container body 2 and an outer surface 33, facing, in use, externally. The rigid cover 3 comprises furthermore a perimeter edge 31 for the engagement with the edge 21 of the container body 2. To this regard, it will be noted that the cover has, at its lower surface 32, a perimeter recess 34, within which the edge 21 of the rigid container body 2 can be fit inserted, with interference. The edge 31 of the rigid cover 3 encloses externally the edge 21 of the container body 2.

The second perimeter profile 7 of the mould according to the disclosure is provided on the lower surface 32 of the rigid cover 3, which lower surface 32 is partially insertable in the container body itself, with such perimeter profile 7 in abutment against the perimeter profile 5 and, accordingly, with the internal shoulder of the container 2.

The rigid cover 3 of the mould according to the disclosure comprises also at least a through opening 35 for the overflow of the bone cement possibly poured in excess in the rigid container body 2. Such a through opening 35 also facilitates the overflow of air bubbles from inside the cavity 4.

The lower surface 32 of the rigid cover 3 comprises a central portion 32a, of substantially rectangular transversal section, extending away from such lower surface 32, and facing, in use, the container body 2.

Now, also the mould 1 according to the second embodiment of the present disclosure comprises weakening means 18 both on the rigid container body 2 and the rigid cover 3 (indicated in Figure 6 merely as a way of example with dashed lines), so that they can both be separated in several parts in order to allow extraction of the spacer device formed therein.

Such weakening means, illustrated schematically in the drawing, comprise at least a reduced thickness line, at which the rigid container body 2 and the rigid cover 3 can be separated in several parts. Such a reduced thickness line is provided with transversally and/or longitudinally both on the rigid container body 2 and on the rigid cover 3.

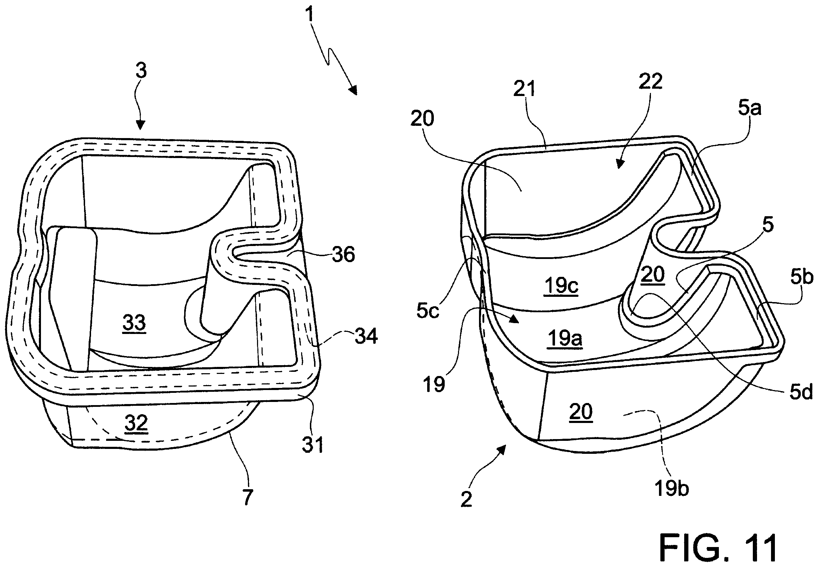

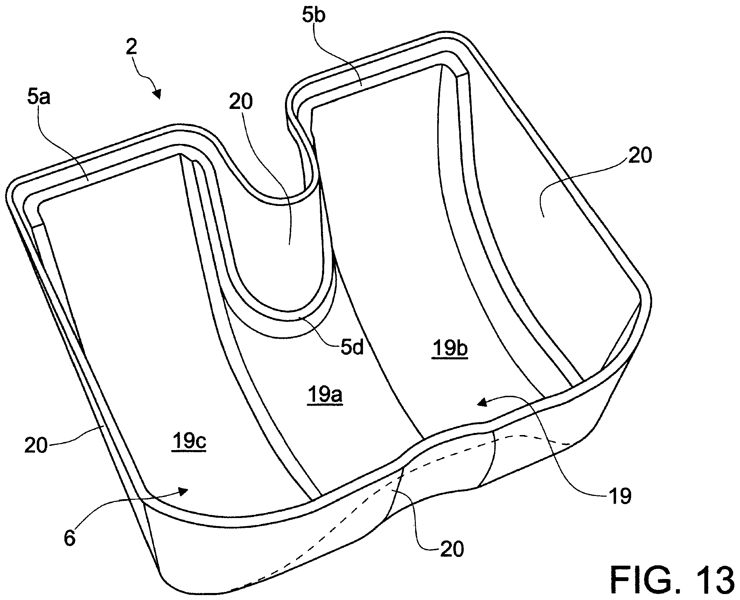

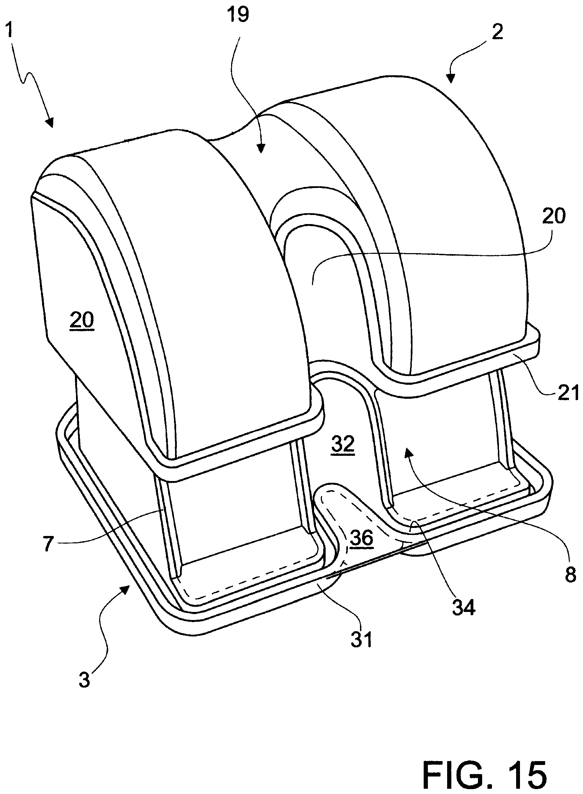

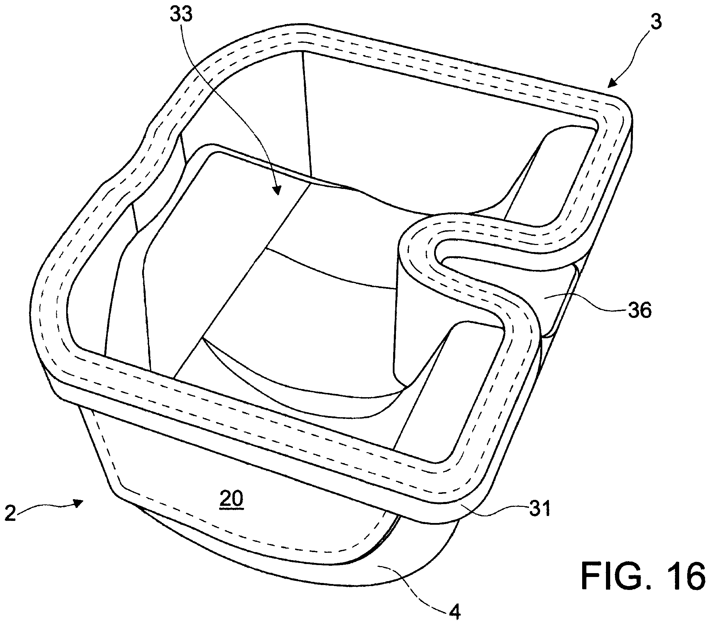

The annexed Figures 11 to 16 show the mould for the forming of a joint spacer according to the third embodiment of the present disclosure, in particular suitable for forming a component or femoral part of a knee joint spacer device . Such a mould comprises at least a rigid container body 2 and at least a rigid cover 3, which can be coupled with each other to delimit a cavity 4 (Figure 16) corresponding to the external configuration of such component or femoral part.

More particularly, the rigid container body 2 of the mould according to the invention has a substantially "C"-shaped plan configuration and circumscribes a bottom wall 19 from which side walls 20 rise. The side walls 20 end at the top with an edge 21 at an upper access opening 22.

The bottom wall 19 of the container body 2 of the mould 1 according to the third embodiment of this invention is substantially concave and has a central portion 19a and two end portions 19b and 19c, at the free ends of "C". The central portion 19a is substantially in relief with respect to the end portions 19b and 19c.

The rigid container body 2 is provided with at least a first perimeter profile 5, which upperly delimits at least a first moulding surface 6 intended to shape at least a first part of the joint spacer device.

The first perimeter profile 5 is defined by an internal shoulder, at the side walls 20 of the rigid container body 2, at a not constant distance from the edge 21 and, therefore, from the bottom wall 19.

More particularly, the perimeter profile 5 of the container body 2 is close to the edge 21 of the container itself at the free ends of the "C" (abutment portions indicated in the figures with the references 5a and 5b) and at an opposite portion, intermediate with respect to such ends (indicated in the figures as 5c). The perimeter profile 5 descends towards the bottom wall 19 of the container body at the remaining end portions 19b and 19c, and at the portion of the side wall 20 comprised between the free ends of "C" (stretch 5d). The remaining portions of the perimeter profile 5 are continuous connection parts, since the perimeter profile 5 is continuous along the side wall 20 of the container 2.

The rigid cover 3 has a substantially "C"-shaped plan configuration and is provided with at least a second perimeter profile 7, delimiting at least a second moulding surface 8, intended to shape at least a second part of such joint spacer device.

The rigid container body 2 and the rigid cover 3 are engageable in a removable manner with each other at respective perimeter profiles 5 and 7, so that the cavity 4 remains delimited therebetween.

Now, with reference to the rigid cover 3 of the mould of Figures 11 to 16, it will be noted how this has a lower surface 32, facing, in use, the respective rigid container body 2 and an outer surface 33, facing, in use, externally. The rigid cover 3 comprises also a perimeter edge 31 for the engagement with the edge 21 of the container body 2. To this regard it will be noted that the rigid cover 3 has, at its lower surface 32, a perimeter recess 34, within which the edge 21 of the rigid container body 2 can be fit inserted, with interference. The edge 31 of the rigid cover 3 encloses externally the edge 21 of the container body 2.

The second perimeter profile 7 of the mould according to the disclosure is provided with on the lower surface 32 of the rigid cover 3, which can be inserted in the container body itself with the perimeter section 7 in abutment against the perimeter profile 5, i.e. with the internal shoulder of the rigid container 2. The second perimeter profile 7 of the cover is therefore configured in a corresponding manner to the respective perimeter profile 5.

The cover 3 of the mould according to the disclosure comprises also at least a through opening (not illustrated in this case in the drawings) for the overflow of the bone cement cast possibly in excess in the rigid container body 2. Such a through opening serves, as previously mentioned with reference to the second embodiment of the present disclosure, to facilitate the overflow of air bubbles from inside the cavity 4.

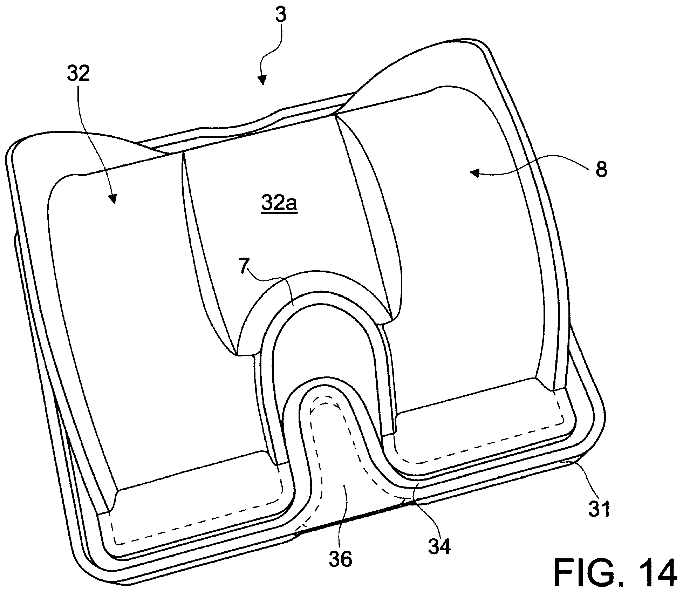

The lower surface 32 of the rigid cover 3 is substantially convex (see in particular Figure 14) and comprises a central portion 32a, recessed with respect to the portions at the free ends of "C". Such recessed portion 32a is intended, in use, to be placed on top of the central portion in relief 19a of the of the container body. Now, also the mould 1 according to the third embodiment of this disclosure comprises weakening means 18 both on the container body 2 and the cover 3, so that both can be separated in several parts to allow extraction of the spacer device formed therebetween.

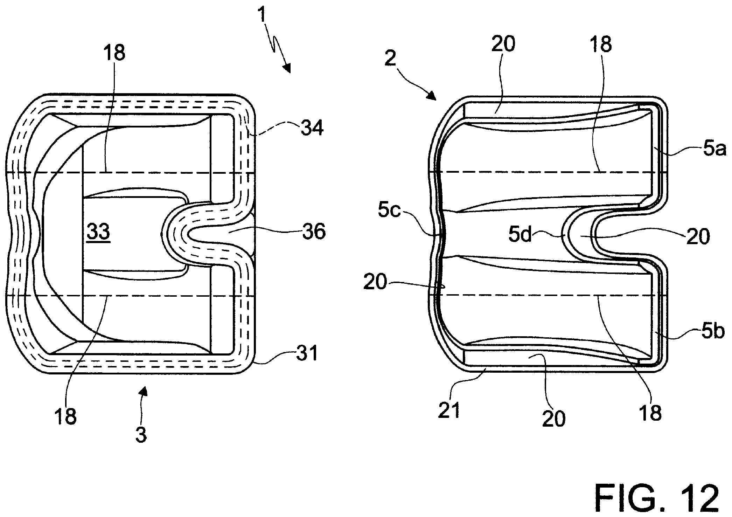

Such weakening means, illustrated schematically in the drawings by a dashed line (for example in Figure 12) comprise at least a reduced thickness line, at which the rigid container body 2 and the rigid cover 3 can be separated in several parts. Such a reduced thickness line is provided with transversally and/or longitudinally, both on the rigid container body 2 and the rigid cover 3.

As for the other embodiments described above, the weakening means 18 are provided with in such a position for which it is possible to extract the joint spacer device or a part thereof from the mould without destroying the mould itself.

Now, with reference to the second and the third embodiment of the present invention, it will be noted that the rigid cover 3 can comprise at least a gripping portion 36, between the free ends of the said "C". Such gripping portion facilitates the application of the rigid cover 3 on the rigid container body 2, as well as their separation in several parts thereof.

Both the rigid container body 2 and the rigid cover 3 can, furthermore, comprise at least a stiffening perimeter rib 37, so that they are elastically undeformable. Such rib is optional and, alternatively thereto, it can be envisaged to make the bottom of the container body 2 or the cover with a greater quantity of material in order to provide greater stiffness to the mould.

Furthermore, once the rigid cover 3 is led to close the rigid container body 2, the mould can be kept closed by additional removable engagement means, not illustrated in the drawings, which comprise, for example, clamp engagement means, or clips or hooks, between the container body 2 and the cover 3.

Once closed, with or without the aid of additional engagement means, the mould can be laid on any of its surface, without the need to make it maintain a precise position during hardening of the bone cement kept therein.

The moulds 1 described above are susceptible to several modifications and variations within the protective scope of the claims that follow.

Thus, for example, if a joint spacer device were to be made for a hip by means of the mould according to the first embodiment of this disclosure, which joint device were equipped of a metal core or any other suitable material able to provide a greater resistance to forces of the spacer device, such mould would comprise also one or more spacer elements (not illustrated in the figures), for example sleeves fitted on such core or element, in any case capable of supporting it, which could be positioned in a predefined position on one or the other moulding surface 6 or 8 and would allow to keep the core of the spacer suitably arranged in the mould, during the forming phase of the joint spacer device.

## Claims

1. A mould for forming a joint spacer device or a part thereof, comprising:
- at least one rigid container body (2) provided with at least one first perimeter profile (5) delimiting at least one first moulding surface (6) intended to shape at least one first portion of said joint spacer or said part thereof;
- at least one rigid cover (3) provided with at least one second perimeter profile (7) delimiting at least one second moulding surface (8) intended to shape at least one second portion of said joint spacer or said part thereof,
said at least one rigid container body (2) and said at least one rigid cover (3) being engageable in a removable manner to each other, at said first and said second perimeter profile (5, 7), so as to delimit a cavity (4) corresponding to the external configuration of said joint spacer or said part thereof
said mould comprising weakening means (18) on said at least one rigid container body (2) and on said rigid cover (3) so that both said at least one rigid container body (2) and said at least one rigid cover (3) are separable into parts to allow the extraction of said at least one spacer device or said at least one part thereof, moulded between them,
wherein said at least one first moulding surface (6) is provided to form a longitudinal half of said at least one spacer device and said at least one second moulding surface (8) is provided to form the other longitudinal half of said at least one spacer device,
wherein said first and/or said second moulding surface (6, 8) is/are formed in a respective base (12, 13), said at least one first and/or second perimeter profile (5, 7) extending substantially orthogonally to and away from said respective base (12, 13),
**characterized in that** said mould comprises at least one perimeter wall having an inclined plane (12a, 13a) between said at least one first and/or second perimeter profile (5,7) and the respective base (12, 13).

2. A mould for forming a joint spacer device or a part thereof, comprising:
- at least one rigid container body (2) provided with at least one first perimeter profile (5) delimiting at least one first moulding surface (6) intended to shape at least one first portion of said joint spacer or said part thereof;
- at least one rigid cover (3) provided with at least one second perimeter profile (7) delimiting at least one second moulding surface (8) intended to shape at least one second portion of said joint spacer or said part thereof,
said mould comprising weakening means (18) on said at least one rigid container body (2) and on said rigid cover (3) so that both said at least one rigid container body (2) and said at least one rigid cover (3) are separable into parts to allow the extraction of said at least one spacer device or said at least one part thereof, moulded between them, and
wherein said at least one rigid container body (2) has an edge (21) delimiting at least one top access opening (22) and wherein said first perimeter profile (5) is a shoulder located internally to said rigid container body (2),
**characterised in that** said at least one rigid container body (2) and said at least one rigid cover (3) being engageable in a removable manner to each other, at said first and said second perimeter profile (5, 7), so as to delimit a cavity (4) corresponding to the external configuration of said joint spacer or said part thereof.

3. A mould according to claim 1 or 2, wherein said weakening means (18) on said at least one rigid container body (2) and on said rigid cover (3) comprise at least a line with reduced thickness, so that said mould is separable into multiple portions at said at least one line with reduced thickness.

4. A mould according to any one of previous claims, wherein said at least one line with reduced thickness is provided transversally and/or longitudinally on said rigid container body (2) and on said rigid cover (3).

5. A mould according to any claim 1 to 4, comprising removable engagement means (14) between said at least one rigid container body (2) and said at least one rigid cover (3) of the male (15) and female (16) type.

6. A mould according to claim 5, wherein said removable engagement means (14) are provided at and along said first perimeter profile (5) and said second perimeter profile (7), so that, when said rigid container body (2) and said at least one respective rigid cover (3) engage each other at their respective perimeter profiles (5, 7), engagement between said male engagement means (15) and said female engagement means (16) of said mould is also achieved.

7. A mould according to any one of previous claims whe dependent from claim 1, wherein each moulding surface (6, 8) comprises a stem portion (9, 9'), a head portion (10, 10') and a connection portion (11, 11') between said stem portion and said head portion.

8. A mould according to any one of previous claims , wherein said at least one rigid container body (2) and said at least one rigid cover (3) are hinged together at their respective bases (12, 13).

9. A mould according to any one of previous claims, wherein said rigid container body (2) and said at least one rigid cover (3) are provided with at least one transversal stiffening rib (17), so that said at least one mould is elastically undeformable.

10. A mould according to any one of previous claims when dependent from claim 2, wherein said at least one rigid cover (3) has a lower in use surface (32), and an upper in use or outer surface (33), as well as a perimeter edge (31) for engagement with said edge (21) of said rigid container body (2), and is configured so that said lower in use surface (32) is insertable in said at least one rigid container body (2), with its second perimeter profile (7) in abutment against said at least one internal shoulder (5).

11. A mould according to any one of previous claims when dependent from claim 2, wherein said second perimeter profile (7) is provided on said lower in use surface (32) of said at least one rigid cover (3).

12. A mould according to any one of previous claims when dependent from claim 2, wherein on said lower in use surface (32) of said rigid cover (3) at least one perimeter recess (34) is formed, so that said edge (21) of said rigid container body (2) can be fit inserted, with interference, in said perimeter recess (34).

13. A mould according to any one of previous claims when dependent from claim 2, wherein said rigid cover (3) comprises at least one through opening (35).

14. A mould according to any one of previous claims when dependent from claim 2, wherein said internal shoulder (5) is provided inside said at least one rigid container body (2) at a substantially constant distance from said edge (21).

15. A mould according to any one of previous claims when dependent from claim 2, wherein said at least one rigid container body (2) has a substantially "C"-shape plan configuration and comprises a bottom wall (19) substantially convex with a central portion (19a) and two end portions (19b, 19c), said central portion (19a) being substantially in low relief with respect to said end portions (19b, 19c).

16. A mould according to any one of previous claims when dependent from claim 2, wherein said rigid cover (3) comprises at least one central portion (32a) having a substantially rectangular cross section, protruding with respect to said lower in use surface (32), and facing, in use, said rigid container body (2).

17. A mould according to any of previous claims when dependent from claim 2, wherein said internal shoulder (5) is provided inside said at least one rigid container body at a substantially not constant distance with respect to said edge (21).

18. A mould according to claim 17, wherein said first perimeter profile (5) comprises at least one portion (5a, 5b) which is close to said edge (21) of said rigid container body (2), at the free ends of the "C" as well as at an intermediate section (5c) opposite to said ends.

19. A mould according to claim 17 or 18, wherein said first perimeter profile (5) is provided close to said bottom wall (19) at the remaining end portions (19b, 19c), as well as at one portion (5c) of said side walls (20) between said free ends of said "C".

20. A mould according to any one of previous claims when dependent from claim 2 or 17 to 19, wherein said at least one rigid container body (2) has a substantially "C"-shape plan configuration, and comprises a substantially concave bottom wall (19) with a central portion (19a) and two end portions (19b, 19c), said central portion (19a) being substantially in relief with respect to said end portions (19b, 19c).

21. A mould according to any one of previous claims when dependent from claim 2 or 17 to 20, wherein said rigid cover (3) comprises, at the lower surface (32) thereof, at least one central portion (32a) and two end portions, said central portion (32a) being recessed with respect to said end portions.

22. A mould according to any one of previous claims when dependent from claim 2, wherein said rigid cover (3) comprises at least one gripping portion (36) between the free ends of said "C".

23. A mould according to any one of previous claims when dependent from claim 2, wherein said rigid container body (2) comprises at least one perimeter stiffening rib (37), so that said at least one rigid container body (2) is elastically undeformable.

24. A mould according to any claim 1 to 23, comprising clamp-type additional removable engagement means between said at least one rigid container body (2) and said at least one rigid cover (3).

## Patentansprüche

1. Form zum Bilden einer Gelenkspacer-Vorrichtung oder eines Teils davon, umfassend:
- mindestens einen starren Behälterkörper (2), der mit mindestens einem ersten Umfangsprofil (5) versehen ist, das mindestens eine erste Formoberfläche (6) begrenzt, die dazu bestimmt ist, mindestens einen ersten Abschnitt des besagten Gelenkspacers oder des besagten Teils davon zu bilden;
- mindestens eine starre Abdeckung (3), die mit mindestens einem zweiten Umfangsprofil (7) versehen ist, das mindestens eine zweite Formoberfläche (8) begrenzt, die dazu bestimmt ist, mindestens einen zweiten Abschnitt des besagten Gelenkspacers oder des besagten Teils davon zu bilden;
wobei der besagte mindestens eine starre Behälterkörper (2) und die besagte mindestens eine starre Abdeckung (3) an dem besagten ersten und dem besagten zweiten Umfangsprofil (5, 7) auf lösbare Weise miteinander in Eingriff bringbar sind, um eine Kavität (4) zu begrenzen, die der äußeren Konfiguration des besagten Gelenkspacers oder des besagten Teils davon entspricht, wobei die besagte Form Schwächungsmittel (18) an dem besagten mindestens einen starren Behälterkörper (2) und an der besagten starren Abdeckung (3) umfasst, sodass sowohl der besagte mindestens eine starre Behälterkörper (2) als auch die besagte mindestens eine starre Abdeckung (3) in Teile trennbar sind, um das Ausziehen der besagten mindestens einen Abstandsvorrichtung oder des besagten mindestens einen Teils davon zu ermöglichen, die zwischen ihnen geformt ist,
worin die besagte mindestens eine erste Formoberfläche (6) vorgesehen ist, um eine Längshälfte der besagten mindestens einen Abstandsvorrichtung zu bilden, und die besagte mindestens eine zweite Formoberfläche (8) vorgesehen ist, um die andere Längshälfte der besagten mindestens einen Abstandsvorrichtung zu bilden,
worin die besagte erste und/oder zweite Formoberfläche (6, 8) in einer jeweiligen Basis (12, 13) ausgebildet ist/sind, wobei sich das besagte mindestens eine erste und/oder zweite Umfangsprofil (5, 7) im Wesentlichen orthogonal zu und von der besagten jeweiligen Basis (12, 13) weg erstreckt,
**dadurch gekennzeichnet, dass** die besagte Form mindestens eine Umfangswand umfasst, die eine geneigte Ebene (12a, 13a) zwischen dem besagten mindestens einen ersten und/oder zweiten Umfangsprofil (5, 7) und der jeweiligen Basis (12, 13) aufweist.

2. Form zum Bilden einer Gelenkspacer-Vorrichtung oder eines Teils davon, umfassend:
- mindestens einen starren Behälterkörper (2), der mit mindestens einem ersten Umfangsprofil (5) versehen ist, das mindestens eine erste Formoberfläche (6) begrenzt, die dazu bestimmt ist, mindestens einen ersten Abschnitt des besagten Gelenkspacers oder des besagten Teils davon zu bilden;
- mindestens eine starre Abdeckung (3), die mit mindestens einem zweiten Umfangsprofil (7) versehen ist, das mindestens eine zweite Formoberfläche (8) begrenzt, die dazu bestimmt ist, mindestens einen zweiten Abschnitt des besagten Gelenkspacers oder des besagten Teils davon zu bilden;
wobei die besagte Form Schwächungsmittel (18) an dem besagten mindestens einen starren Behälterkörper (2) und an der besagten starren Abdeckung (3) umfasst, sodass sowohl der besagte mindestens eine starre Behälterkörper (2) als auch die besagte mindestens eine starre Abdeckung (3) in Teile trennbar sind, um das Ausziehen der besagten mindestens einen Abstandsvorrichtung oder des besagten mindestens einen Teils davon zu ermöglichen, die zwischen ihnen geformt ist, und
worin der besagte mindestens eine starre Behälterkörper (2) eine Kante (21) aufweist, die mindestens eine obere Zugangsöffnung (22) begrenzt, und worin das besagte erste Umfangsprofil (5) eine Schulter ist, die innerhalb des besagten starren Behälterkörpers (2) angeordnet ist,
**dadurch gekennzeichnet, dass** der besagte mindestens eine starre Behälterkörper (2) und die besagte mindestens eine starre Abdeckung (3) an dem besagten ersten und dem besagten zweiten Umfangsprofil (5, 7) auf lösbare Weise miteinander in Eingriff bringbar sind, um eine Kavität (4) zu begrenzen, die der äußeren Konfiguration des besagten Gelenkspacers oder des besagten Teils davon entspricht.

3. Form nach Anspruch 1 oder 2, worin die besagten Schwächungsmittel (18) an dem besagten mindestens einen starren Behälterkörper (2) und an der besagten starren Abdeckung (3) mindestens eine Linie mit reduzierter Dicke umfassen, sodass die besagte Form in mehrere Abschnitte an der besagten mindestens einen Linie mit reduzierter Dicke trennbar ist.

4. Form nach irgendeinem der vorangegangenen Ansprüche, worin die besagte mindestens eine Linie mit reduzierter Dicke quer und/oder längs an dem besagten starren Behälterkörper (2) und an der besagten starren Abdeckung (3) vorgesehen ist.

5. Form nach irgendeinem der Ansprüche 1 bis 4, umfassend lösbare Eingreifmittel (14) zwischen dem besagten mindestens einen starren Behälterkörper (2) und der besagten mindestens einen starren Abdeckung (3) des männlichen (15) und weiblichen (16) Typs.

6. Form nach Anspruch 5, worin die besagten lösbaren Eingreifmittel (14) an und entlang des besagten ersten Umfangsprofils (5) und des besagten zweiten Umfangsprofils (7) vorgesehen sind, sodass, wenn der besagte starre Behälterkörper (2) und die besagte mindestens eine jeweilige starre Abdeckung (3) an ihren jeweiligen Umfangsprofilen (5, 7) miteinander in Eingriff stehen, auch ein Eingriff zwischen den besagten männlichen Eingreifmitteln (15) und den besagten weiblichen Eingreifmitteln (16) der besagten Form erreicht wird.

7. Form nach irgendeinem der vorangegangenen Ansprüche, wenn abhängig von Anspruch 1, worin jede Formoberfläche (6, 8) einen Schaftabschnitt (9, 9'), einen Kopfabschnitt (10, 10') und einen Verbindungsabschnitt (11, 11') zwischen dem besagten Schaftabschnitt und dem besagten Kopfabschnitt umfasst.

8. Form nach irgendeinem der vorangegangenen Ansprüche, worin der besagte mindestens eine starre Behälterkörper (2) und die besagte mindestens eine starre Abdeckung (3) an ihren jeweiligen Basen (12, 13) gelenkig miteinander verbunden sind.

9. Form nach irgendeinem der vorangegangenen Ansprüche, worin der besagte starre Behälterkörper (2) und die besagte mindestens eine starre Abdeckung (3) mit mindestens einer queren Versteifungsrippe (17) versehen sind, sodass die besagte mindestens eine Form elastisch unverformbar ist.

10. Form nach irgendeinem der vorangegangenen Ansprüche, wenn abhängig von Anspruch 2, worin die besagte mindestens eine starre Abdeckung (3) eine bei Verwendung untere Oberfläche (32) und eine bei Verwendung obere oder äußere Oberfläche (33) sowie eine Umfangskante (31) zum Eingriff mit der besagten Kante (21) des besagte starren Behälterkörpers (2) aufweist und so konfiguriert ist, dass die besagte bei Verwendung untere Oberfläche (32) in den besagten mindestens einen starren Behälterkörper (2) einsetzbar ist, wobei ihr zweites Umfangsprofil (7) an der besagten mindestens einen Innenschulter (5) anliegt.

11. Form nach irgendeinem der vorangegangenen Ansprüche, wenn abhängig von Anspruch 2, worin das besagte zweite Umfangsprofil (7) auf der besagten bei Verwendung unteren Oberfläche (32) der besagten mindestens einen starren Abdeckung (3) vorgesehen ist.

12. Form nach irgendeinem der vorangegangenen Ansprüche, wenn abhängig von Anspruch 2, worin auf der besagten bei Verwendung unteren Oberfläche (32) der besagten starren Abdeckung (3) mindestens eine Umfangsaussparung (34) ausgebildet ist, sodass die besagte Kante (21) des besagten starren Behälterkörpers (2) in die besagte Umfangsaussparung (34) mit Eingriff passend eingesetzt werden kann.

13. Form nach irgendeinem der vorangegangenen Ansprüche, wenn abhängig von Anspruch 2, worin die besagte starre Abdeckung (3) mindestens eine Durchgangsöffnung (35) umfasst.

14. Form nach irgendeinem der vorangegangenen Ansprüche, wenn abhängig von Anspruch 2, worin die besagte Innenschulter (5) innerhalb des besagten mindestens einen starren Behälterkörpers (2) in einem im Wesentlichen konstanten Abstand von der besagten Kante (21) vorgesehen ist.

15. Form nach irgendeinem der vorangegangenen Ansprüche, wenn abhängig von Anspruch 2, worin der besagte mindestens eine starre Behälterkörper (2) eine im Wesentlichen "C"-förmige Draufsichtkonfiguration hat und eine Bodenwand (19) umfasst, die mit einem mittleren Abschnitt (19a) und zwei Endabschnitten (19b, 19c) im Wesentlichen konvex ist, wobei der besagte mittlere Abschnitt (19a) in Bezug auf die besagten Endabschnitte (19b, 19c) im Wesentlichen in flachem Relief ausgebildet ist.

16. Form nach irgendeinem der vorangegangenen Ansprüche, wenn abhängig von Anspruch 2, worin die besagte starre Abdeckung (3) mindestens einen mittleren Abschnitt (32a) mit einem im Wesentlichen rechteckigen Querschnitt umfasst, der in Bezug auf die besagte bei Verwendung untere Oberfläche (32) vorsteht und, bei Verwendung, dem besagten starren Behälterkörper (2) zugewandt ist.

17. Form nach irgendeinem der vorangegangenen Ansprüche, wenn abhängig von Anspruch 2, worin die besagte Innenschulter (5) innerhalb des besagten mindestens einen starren Behälterkörpers in einem im Wesentlichen nicht konstanten Abstand in Bezug auf die besagte Kante (21) vorgesehen ist.

18. Form nach Anspruch 17, worin das besagte erste Umfangsprofil (5) mindestens einen Abschnitt (5a, 5b) umfasst, der nahe an der besagten Kante (21) des besagten starren Behälterkörpers (2), an den freien Enden des "C"s sowie an einem den besagten Enden gegenüberliegenden Zwischenabschnitt (5c) liegt.

19. Form nach Anspruch 17 oder 18, worin das besagte erste Umfangsprofil (5) nahe der besagten Bodenwand (19) an den verbleibenden Endabschnitten (19b, 19c) sowie an einem Abschnitt (5c) der besagten Seitenwände (20) zwischen den besagten freien Enden des besagten "C"s vorgesehen ist.

20. Form nach irgendeinem der vorangegangenen Ansprüche, wenn abhängig von Anspruch 2 oder 17 bis 19, worin der besagte mindestens eine starre Behälterkörper (2) eine im Wesentlichen "C"-förmige Draufsichtkonfiguration hat und eine im Wesentlichen konkave Bodenwand (19) mit einem mittleren Abschnitt (19a) und zwei Endabschnitten (19b, 19c) umfasst, wobei der besagte mittlere Abschnitt (19a) in Bezug auf die besagten Endabschnitte (19b, 19c) im Wesentlichen in flachem Relief ausgebildet ist.

21. Form nach irgendeinem der vorangegangenen Ansprüche, wenn abhängig von Anspruch 2 oder 17 bis 20, worin die besagte starre Abdeckung (3) an ihrer unteren Oberseite (32) mindestens einen mittleren Abschnitt (32a) und zwei Endabschnitte umfasst, wobei der besagte mittlere Abschnitt (32a) in Bezug auf die besagten Endabschnitte ausgespart ist.

22. Form nach irgendeinem der vorangegangenen Ansprüche, wenn abhängig von Anspruch 2, worin die besagte starre Abdeckung (3) mindestens einen Greifabschnitt (36) zwischen den freien Enden des besagten "C"s umfasst.

23. Form nach irgendeinem der vorangegangenen Ansprüche, wenn abhängig von Anspruch 2, worin der besagte starre Behälterkörper (2) mindestens eine Umfangsversteifungsrippe (37) umfasst, sodass der besagte mindestens eine starre Behälterkörper (2) elastisch unverformbar ist.

24. Form nach irgendeinem der Ansprüche 1 bis 23, umfassend klemmenartige zusätzliche lösbare Eingreifmittel zwischen dem besagten mindestens einen starren Behälterkörper (2) und der besagten mindestens einen starren Abdeckung (3).

## Revendications

1. Moule pour former une entretoise d'articulation ou une partie de celle-ci, comprenant :
- au moins un corps de récipient rigide (2) doté d'au moins un premier profil périmétrique (5) délimitant au moins une première surface de moulage (6) destinée à former au moins une première portion de ladite entretoise d'articulation ou de ladite partie de celle-ci ;
- au moins un couvercle rigide (3) doté d'au moins un deuxième profil périmétrique (7) délimitant au moins une deuxième surface de moulage (8) destinée à former au moins une deuxième portion de ladite entretoise d'articulation ou de ladite partie de celle-ci,
ledit au moins un corps de récipient rigide (2) et ledit au moins un couvercle rigide (3) pouvant être accouplés de manière amovible l'un avec l'autre, au niveau dudit premier et dudit deuxième profils périmétriques (5, 7) de façon à délimiter une cavité (4) correspondant à la configuration externe de ladite entretoise d'articulation ou de ladite partie de celle-ci
ledit moule comprenant des moyens d'affaiblissement (18) sur ledit au moins un corps de récipient rigide (2) et sur ledit couvercle rigide (3) de telle sorte que ledit au moins un corps de récipient rigide (2) et ledit au moins un couvercle rigide (3) soient séparables en parties pour permettre l'extraction de ladite au moins une entretoise ou ladite au moins une partie de celle-ci, moulée entre elles,
dans lequel ladite au moins une première surface de moulage (6) est prévue pour former une moitié longitudinale de ladite au moins une entretoise et ladite au moins une deuxième surface de moulage (8) est prévue pour former l'autre moitié longitudinale de ladite au moins une entretoise,
dans lequel ladite première et/ou ladite deuxième surface de moulage (6, 8) est/sont formées dans une base (12, 13) respective, ledit au moins un premier et/ou deuxième profil périmétrique (5, 7) s'étendant sensiblement orthogonalement par rapport et loin de ladite base (12, 13) respective,
**caractérisé en ce que** ledit moule comprend au moins une paroi périmétrique ayant un plan incliné (12a, 13a) entre ledit au moins un premier et ou deuxième profils périmétriques (5,7) et la base (12, 13) respective.

2. Moule pour former une entretoise d'articulation ou une partie de celle-ci, comprenant :
- au moins un corps de récipient rigide (2) doté d'au moins un premier profil périmétrique (5) délimitant au moins une première surface de moulage (6) destinée à former au moins une première portion de ladite entretoise d'articulation ou de ladite partie de celle-ci ;
- au moins un couvercle rigide (3) doté d'au moins un deuxième profil périmétrique (7) délimitant au moins une deuxième surface de moulage (8) destinée à former au moins une deuxième portion de ladite entretoise d'articulation ou de ladite partie de celle-ci,
ledit moule comprenant des moyens d'affaiblissement (18) sur ledit au moins un corps de récipient rigide (2) et sur ledit couvercle rigide (3) de telle sorte que ledit au moins un corps de récipient rigide (2) et ledit au moins un couvercle rigide (3) soient séparables en parties pour permettre l'extraction de ladite au moins une entretoise ou ladite au moins une partie de celle-ci, moulée entre elles, et
dans lequel ledit au moins un corps de récipient rigide (2) comporte un bord (21) délimitant au moins une ouverture d'accès supérieure (22) et dans lequel ledit premier profil périmétrique (5) est un épaulement situé à l'intérieur dudit corps de récipient rigide (2), **caractérisé en ce que** ledit au moins un corps de récipient rigide (2) et ledit au moins un couvercle rigide (3) pouvant être accouplés de manière amovible l'un avec l'autre, au niveau dudit premier et dudit deuxième profils périmétriques (5, 7) de façon à délimiter une cavité (4) correspondant à la configuration externe de ladite entretoise d'articulation ou de ladite partie de celle-ci.

3. Moule selon la revendication 1 ou 2, dans lequel lesdits moyens d'affaiblissement (18) sur ledit au moins un corps de récipient rigide (2) et sur ledit couvercle rigide (3) comprennent au moins une ligne d'épaisseur réduite, de telle sorte que ledit moule soit séparable en plusieurs portions sur ladite au moins une ligne d'épaisseur réduite.

4. Moule selon l'une quelconque des revendications précédentes, dans lequel ladite au moins une ligne d'épaisseur réduite est disposée transversalement et/ou longitudinalement sur ledit corps de récipient rigide (2) et sur ledit couvercle rigide (3).

5. Moule selon l'une quelconque des revendications 1 à 4, comprenant des moyens d'accouplement amovibles (14) entre ledit au moins un corps de récipient rigide (2) et ledit au moins un couvercle rigide (3) du type mâle (15) et femelle (16).

6. Moule selon la revendication 5, dans lequel lesdits moyens d'accouplement amovibles (14) sont disposés au niveau et le long dudit premier profil périmétrique (5) et dudit deuxième profil périmétrique (7), de telle sorte que, lorsque ledit corps de récipient rigide (2) et ledit au moins un respectif couvercle rigide (3) s'accouplent l'un à l'autre au niveau de leurs profils périmétriques (5, 7) respectifs, l'accouplement entre lesdits moyens d'accouplement mâles (15) et lesdits moyens d'accouplement femelles (16) dudit moule est également obtenu.

7. Moule selon l'une quelconque des revendications précédentes quand elle dépend de la revendication 1, dans lequel chaque surface de moulage (6, 8) comprend une portion de tige (9, 9'), une portion de tête (10, 10') et une portion de raccordement (11, 11') entre ladite portion de tige et ladite portion de tête.

8. Moule selon l'une quelconque des revendications précédentes, dans lequel ledit au moins un corps de récipient rigide (2) et ledit au moins un couvercle rigide (3) sont articulés ensemble au niveau de leurs bases (12, 13) respectives.

9. Moule selon l'une quelconque des revendications précédentes, dans lequel ledit corps de récipient rigide (2) et ledit au moins un couvercle rigide (3) sont dotés d'au moins une nervure de rigidification transversale (17), de telle sorte que ledit au moins un moule soit indéformable élastiquement.

10. Moule selon l'une quelconque des revendications précédentes quand elle dépend de la revendication 2, dans lequel ledit au moins un couvercle rigide (3) comporte une surface inférieure en utilisation (32), et une surface supérieure en utilisation ou extérieure (33), ainsi qu'un bord périmétrique (31) pour l'accouplement avec ledit bord (21) dudit corps de récipient rigide (2), et est configuré de telle sorte que ladite surface inférieure en utilisation (32) puisse être insérée dans ledit au moins un corps de récipient rigide (2), avec son deuxième profil périmétrique (7) en butée contre ledit au moins un épaulement interne (5).

11. Moule selon l'une quelconque des revendications précédentes quand elle dépend de la revendication 2, dans lequel ledit deuxième profil périmétrique (7) est disposé sur ladite surface inférieure en utilisation (32) dudit au moins un couvercle rigide (3).

12. Moule selon l'une quelconque des revendications précédentes quand elle dépend de la revendication 2, dans lequel sur ladite surface inférieure en utilisation (32) dudit couvercle rigide (3) au moins un renfoncement périmétrique (34) est formé, de telle sorte que ledit bord (21) dudit corps de récipient rigide (2) puisse être inséré par ajustement, avec interférence, dans ledit renfoncement périmétrique (34) .

13. Moule selon l'une quelconque des revendications précédentes quand elle dépend de la revendication 2, dans lequel ledit couvercle rigide (3) comprend au moins une ouverture traversante (35).

14. Moule selon l'une quelconque des revendications précédentes quand elle dépend de la revendication 2, dans lequel ledit épaulement interne (5) est disposé à l'intérieur dudit au moins un corps de récipient rigide (2) à une distance sensiblement constante dudit bord (21) .

15. Moule selon l'une quelconque des revendications précédentes quand elle dépend de la revendication 2, dans lequel ledit au moins un corps de récipient rigide (2) a une forme plane sensiblement en « C » et comprend une paroi inférieure (19) sensiblement convexe avec une portion centrale (19a) et deux portions d'extrémité (19b, 19c), ladite portion centrale (19a) étant sensiblement en bas relief par rapport auxdites portions d'extrémité (19b, 19c).

16. Moule selon l'une quelconque des revendications précédentes quand elle dépend de la revendication 2, dans lequel ledit couvercle rigide (3) comprend au moins une portion centrale (32a) ayant une section transversale sensiblement rectangulaire, saillante par rapport à ladite surface inférieure en utilisation (32), et orientée pendant l'utilisation vers ledit corps de récipient rigide (2).

17. Moule selon l'une quelconque des revendications précédentes quand elle dépend de la revendication 2, dans lequel ledit épaulement interne (5) est disposé à l'intérieur dudit au moins un corps de récipient rigide à une distance sensiblement non constante dudit bord (21) .

18. Moule selon la revendication 17, dans lequel ledit premier profil périmétrique (5) comprend au moins une portion (5a, 5b) proche dudit bord (21) dudit corps de récipient rigide (2), aux extrémités libres de la forme en « C » ainsi qu'à une section intermédiaire (5c) opposée auxdites extrémités.

19. Moule selon la revendication 17 ou 18, dans lequel ledit premier profil périmétrique (5) est agencé à proximité de ladite paroi inférieure (19) sur les portions d'extrémité restantes (19b, 19c), ainsi qu'au niveau d'une portion (5c) desdites parois latérales (20) entre lesdites extrémités libres de ladite forme en « C ».

20. Moule selon l'une quelconque des revendications précédentes quand elle dépend de la revendication 2 ou 17 à 19, dans lequel ledit au moins un corps de récipient rigide (2) a une forme plane sensiblement en « C », et comprend une paroi inférieure (19) sensiblement concave avec une portion centrale (19a) et deux portions d'extrémité (19b, 19c), ladite portion centrale (19a) étant sensiblement en relief par rapport auxdites portions d'extrémité (19b, 19c).

21. Moule selon l'une quelconque des revendications précédentes quand elle dépend de la revendication 2 ou 17 à 20, dans lequel ledit couvercle rigide (3) comprend, sur sa surface inférieure (32), au moins une portion centrale (32a) et deux portions d'extrémité, ladite portion centrale (32a) étant encastrée par rapport auxdites portions d'extrémité.

22. Moule selon l'une quelconque des revendications précédentes quand elle dépend de la revendication 2, dans lequel ledit couvercle rigide (3) comprend au moins une portion de préhension (36) entre les extrémités libres de ladite forme en « C ».

23. Moule selon l'une quelconque des revendications précédentes quand elle dépend de la revendication 2, dans lequel ledit corps de récipient rigide (2) comprend au moins une nervure de rigidification périmétrique (37), de telle sorte que ledit au moins un corps de récipient rigide (2) soit indéformable élastiquement.

24. Moule selon l'une quelconque des revendications 1 à 23, comprenant des moyens d'accouplement supplémentaires de type à bride amovibles entre ledit au moins un corps de récipient rigide (2) et ledit au moins un couvercle rigide (3).
